Europäisches Patentamt

European Patent Office

Office européen des brevets

(19)

(11) Numéro de publication : **0 270 628 B1**

(12)

# FASCICULE DE BREVET EUROPEEN

(45) Date de publication du fascicule du brevet :
**13.05.92 Bulletin 92/20**

(51) Int. Cl.$^5$ : **A61M 5/14**

(21) Numéro de dépôt : **87903889.1**

(22) Date de dépôt : **17.06.87**

(86) Numéro de dépôt international :
**PCT/FR87/00226**

(87) Numéro de publication internationale :
**WO 87/07843 30.12.87 Gazette 87/29**

(54) **DISPOSITIF POUSSE-SERINGUE MONOCOURSE POUR INJECTIONS PARENTERALES AUTOMATIQUES A ASSERVISSEMENT ET PROGRAMMATION.**

(30) Priorité : **18.06.86 FR 8608781**

(43) Date de publication de la demande :
**15.06.88 Bulletin 88/24**

(45) Mention de la délivrance du brevet :
**13.05.92 Bulletin 92/20**

(84) Etats contractants désignés :
**AT BE CH DE FR GB IT LI LU NL SE**

(56) Documents cités :
**EP-A- 0 171 337**
**DE-A- 2 809 990**
**DE-A- 3 307 810**
**US-A- 3 731 679**
**US-A- 3 858 581**

(73) Titulaire : **HAZON, Bernard**
**5 avenue de l'Opéra**
**F-75001 Paris (FR)**

(72) Inventeur : **HAZON, Bernard**
**5 avenue de l'Opéra**
**F-75001 Paris (FR)**

(74) Mandataire : **Portal, Gérard et al**
**Cabinet Beau de Loménie 55, rue**
**d'Amsterdam**
**F-75008 Paris (FR)**

EP 0 270 628 B1

Jouve, 18, rue Saint-Denis, 75001 PARIS

## Description

Le contrôle du débit lors des injections, qu'elles soient intraveineuses, intravasculaires ou sous-cutanées, avec le materiel à usage unique existant, reste aléatoire même entre les mains d'un opérateur entraîné. Ceci est encore plus vrai lorsque, à partir d'une même seringue, on doit délivrer une pluralité de volumes unitaires soit échelonnés dans le temps, soit répartis dans une pluralité de sites. Qu'il s'agisse d'un débit continu ou discontinu, son contrôle ne peut être assuré que par celui de la translation du piston de la seringue. C'est pourquoi de nombreux dispositifs de translation médiate ont été proposés, utilisant en général une vis ou une crémaillère dont le mouvement est controlé soit par lecture directe, soit mieux par asservissement, que ce mécanisme soit entraîné directement par l'opérateur comme dans le brevet Français n° 2 583 291 ou par un servo-mécanisme généralement électrique.

L'utilité de ces dispositifs est particulièrement évidente pour les traitements médicaux dits au long cours comme les administrations répétitives de drogues telles que antimitotiques, antibiotiques, hormones, si l'on ne désire pas multiplier le nombre d'injections, et partant de punctures cutanées.

Dans l'art actuel d'administration de tels traitements médicamenteux, on recourt généralement aux injections intra-veineuses répétitives. Pour ce faire, on institue le plus souvent une perfusion veineuse par gravité au moyen d'un cathéter ou d'une aiguille intra-vasculaire à demeure relié à une tubulure munie de moyens de contrôle du débit et dans laquelle on injecte répétitivement la ou les drogues dans une sone perforable et auto-obturatrice ad'hoc. Cette technique, qui n'a pour but que d'empêcher l'obturation du cathéter par coagulation du sang, présente de nombreux inconvénients, notamment lors les déplacements du patient.

En effet, le maintien d'un gradient de pression constant entre le flacon de perfusion et le système vasculaire est aléatoire, voire impossible, ce qui entraîne des reflux de sang dans la tubulure générateurs de coagulation intempestive et la quasi impossibilité de maintenir un débit de perfusion suffisant.

D'un autre côté, les quantités de solution nécessaires au maintien du cathéter sont importantes, ce qui n'est pas sans inconvénient par suite des surcharges hydriques ou de solutés contenus dans la solution (diabète).

Dans la présente invention, on choisit de substituer au générateur de pression que constitue un flacon en gravité, le générateur de débit qui est constitué par un volume variable, c'est à dire une seringue. Ce générateur de débit permet en outre, au contraire du générateur de pression, les injections intra-artérielles et un meilleur contrôle des perfusions sous-cutanées et intra-dermiques, c'est à dire chaque fois que la contre-pression développée au site d'injection est élevée et incontrôlable.

Il a été proposé de nombreux dispositifs pousse-seringue automatisés, tels creux des brevets:

DE- A - 2 809 990 qui forme le préambule de la revendication 1, US- A- 3 371 679. Ils comportent un mécanisme pousse-piston composé d'un moteur, une vis-sans-fin tournant sur paliers, un chariot débrayable de la vis sans-fin permettant le retour arrière manuel. La vis-sans-fin et le chariot sont apparents en totalité ou en partie. Le boîtier comporte un moyen de contention de la seringue par verrouillage. Le contrôle du débit a été obtenu dans les réalisations les plus anciennes par le contrôle de la vitesse angulaire du moteur.

Plus récemment, et pour étendre la gamme des débits effectivement réalisables, il a été préféré un fonctionnement discontinu du moteur, la vitesse angulaire moyenne pouvant alors varier de 1 à 100. En général, ce résultat est obtenu par un moteur à courant continu dont la rotation est initiée par une horloge et terminée par une came mécaniquement solidaire de la vis-sans-fin et agissant directement sur un contacteur. Dans la mesure où les périodes de relaxation sont courtes en regard de la durée de demie-vie de la drogue injectée, les variations de la concentration systémique de la drogue seront négligeables. La durée totale de vidange de la séringue est une variable qui dépend du volume nominal de la seringue, du débit moyen souhaité et du titre en produit actif de la solution injectée.

L'objet de la présente invention est un dispositif pousse-seringue monocourse électromécanique alimenté par batterie rechargeable, portable et pour traitement ambulatoire, à autonomie de plusieurs heures, comprenant un boîtier parallélipipèdique, un ensemble électromécanique composé d'un moteur à courant continu couplé à un réducteur, une vis-sans-fin entraînant un chariot, et un ensemble de commande électronique, caractérisé en ce que la contention de la seringue est assurée par une pince de section trapézoïdale réalisée par les expansions adéquatement formées des faces latérales du boîtier parallélipipèdique dont la face supérieure libre est fermé par la face supérieure d'une enveloppe-guide quasi étanche et de section polyeduque contenant l'ensemble électro-mécanique, et en ce que le chariot est glissant à l'intérieur de l'enveloppe guide et de section homologue à celle-ci, et en ce que l'élasticité de ladite pince est renforcée par la présence de deux bandeaux portées par les faces latérales du boîtier, et en ce que l'ensemble électro-mécanique est monté flottant à l'intérieur de l'enveloppe-guide et composé d'un moteur à courant continu sans fer tournant à excitation par aimant permanent de faible inertie et d'un réducteur à haut rendement, et en ce que la vis-sans-fin est fixée directement et coaxialement à l'arbre de sortie du réducteur et libre de tout palier en bout, et en ce que les différentes fonctions du dispositif sont contrôlées par un ensemble électronique de programmation et d'asservissement tel que l'angle de rotation de la vis-sans-fin correspondant à un volume prédéterminé est contrôlé par un aimant bipolaire tournant en synchronisme avec la vis sans fin agissant à distance à travers l'enveloppe-guide sur un disrupteur, que la pression du fluide contenu dans la seringue est contrôlée par l'évaluation de la force de réaction exercée par le piston de la seringue sur le chariot au moyen de contrôle de l'intensité traversant l'induit du motoréducteur, que le retour en position de départ du chariot est assuré par l'inversion du sens de rotation du motoréducteur alimenté sous tension maximale jusqu'à ce que le chariot ait atteint ladite position de départ, que le passage à la position de départ interrompt l'alimentation de tous les circuits électriques, à l'exception d'un unique circuit destiné à une initiation de la marche avant, et en ce que une prise de charge accessible de l'extérieur permet la recharge des batteries sans démontage et/ou le contrôle de l'état de décharge de la batterie au moyen d'un dispositif externe.

Dans la présente invention, il a été trouvé que tout le système electromécanique d'avancement du piston pouvait être situé à l'intérieur d'une enveloppe-guide qui sert d'entretoise aux deux faces latérales d'un boîtier qui suffit dès lors à maintenir la seringue, sans adjonction d'un moyen pariculier de contention. L'avantage de cette technique réside dans la grande compacité et miniaturisation du dispositif. Le débit moyen est obtenu par la délivrance d'un nombre fixe de volumes ou bolus injectés à chaque période pendant une durée totale prédéterminée, choisie de telle manière qu'elle coïncide avec avec le nycthémère.

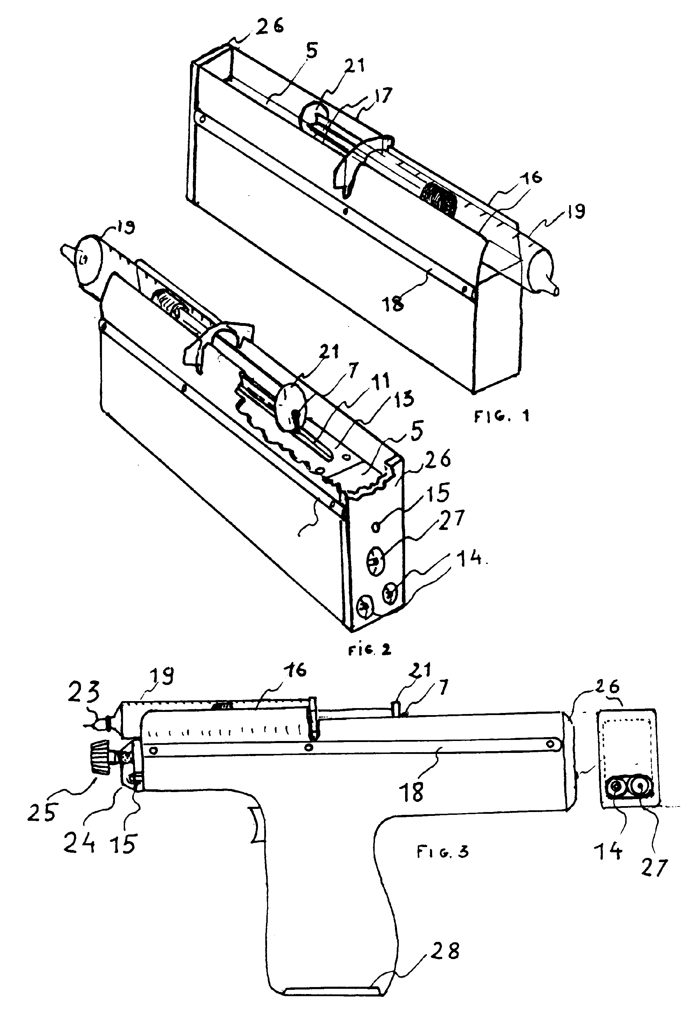

A titre d'exemple non limitatif, nous décrirons en référence aux figures 1 à 7 annexées un dispositif pousse-seringue à volumes unitaires constants programmé par horloge, plus spécialement destiné à l'entretien de cathéters intra-veineux pour traitement ambulatoire.

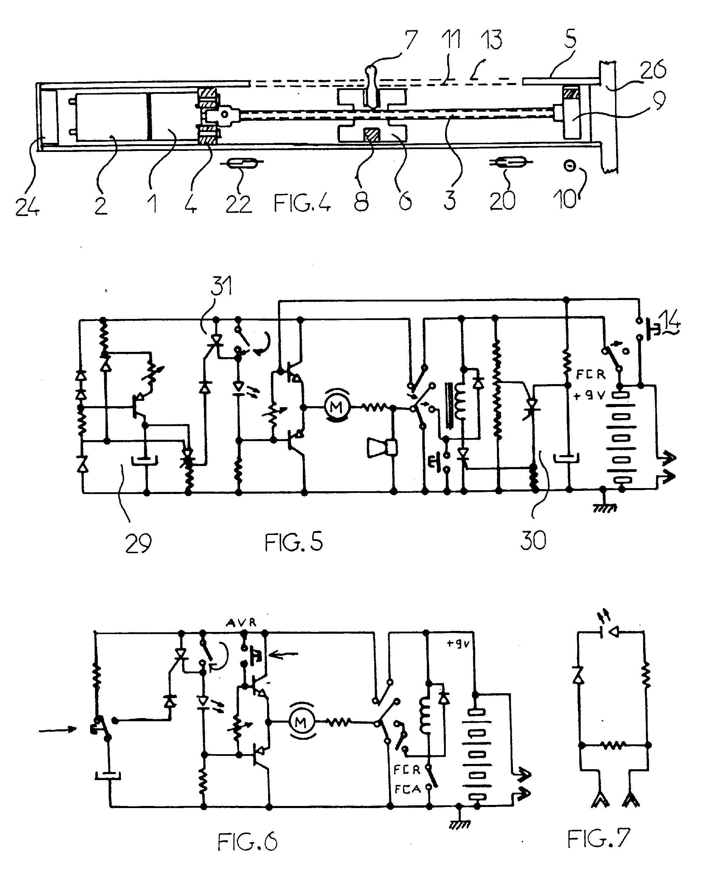

Le dispositif se compose du mécanisme pousse-seringue proprement dit et d'un ensemble de programmation électronique à alimentation indépendante du secteur. Le dispositif pousse-seringue décrit dans la Figure 4 se compose d'un motoréducteur (2-1) avantageusement constitué d'un moteur (2) à courant continu sans fer-tournant à excitation par aimant permanent de faible inertie et d'un réducteur (1) à haut rendement et à arbre de sortie coaxial sur lequel est fixée directement la vis-sans-fin (3) par un manchon rigide. Le motoréducteur (2-1) est fixé par le nez au moyen d'un matériau élastique (4) qui lui permet ainsi qu'à la vis-sans-fin (3) de flotter légèrement à l'intérieur de l'enveloppe-guide (5) pour pallier les inévitables défauts d'alignement de l'ensemble, la vis-sans-fin (3) étant libre de tout palier. Ladite vis-sans-fin (3) se visse et dévisse dans un chariot (6) fileté porteur d'un pion (7) pousse-piston et d'un aimant (8) agissant sur des contacteurs et/ou inverseurs extérieurs à l'enveloppe-guide (5). Ladite vis-sans-fin (3) porte en outre un aimant tournant (9) qui agit sur un autre contacteur (10) sensible au magnètisme et extérieur à l'enveloppe-guide (5). Le guidage du chariot (6) est réalisé par l'enveloppe (5) elle-même qui est à cet effet un profilé de section polyèdrique homologue à celle du chariot (6) La face supérieure de l'enveloppe-guide (5) comporte une fraisure destinée au passage du pion (7) pousse -piston.

Un rideau élastique (11) protège la vis-sans-fin (3) et son chariot (6) concourant à la quasi-étanchéïté de l'enveloppe-guide (5). Ledit rideau (11) peut avantageusement être réalisé dans un matériau fibreux hydrofugé, par exemple un feutre textile, serré par une plaquette (13) vissée contre l'enveloppe guide (5). La face inférieure de l'enveloppe-guide (5) constitue un support et une protection de l'ensemble électronique 45 décrit plus avant et de la batterie d'alimentation.

Le boitier (Fig. 1 et Fig. 2) est construit autour de l'enveloppe-guide (5) qui en constitue l'armature.

Ce boitier a la forme d'un parallélipipède rectangle, aussi bien intérieurement qu'extérieurement. Sa face supérieure est directement formée par la face supérieure de l'enveloppe-guide (5). Sa face postérieure (26) est amovible et porte seule les commandes (14) et affichages (15) de l'appareil. Ses faces latérales sont prolongées au dessus de l'enveloppe-guide (5) par des expansions sur toute la longeur dudit boitier.

Adéquatement formées, ces expansions constituent la pince (16) porte-seringue de section trapezoïdale à la partie avant de l'appareil, et à la partie moyenne les gardes (17) protège-piston.

Deux bandeaux (18) parallèles à l'enveloppe-guide (5) et vissés sur elle à travers la paroi du boitier renforcent l'ensemble et améliorent l'élasticité et la solidité de ladite pince (16) porte-seringue. On obtient ainsi un boitier particulièrement léger et compact pouvant être réalisé par des moyens simples et peu onéreux et dans lequel l'introduction de l'ensemble électro-mécanique (Fig. 4), électronique et porte arrière (26) est particulièrement aisée et rapide.

L'appareil de forme parallélipipédique rectangle, par sa forme géométrique simple et l'excellente protection de la seringue (19) par les expansions du boitier et du mécanisme par l'enveloppe-guide (5) est particulièrement apte au traitement ambulatoire, par exemple en étant porté dans une poche de vêtement ou fixé par un mini-harnais.

Il a été retenu non limitativement un mode principal de logique.

Le dispositif pousse-seringue à horloge délivrant des bolus, plus spécialement destiné au maintien de la perméabilité de cathéters intra-vasculaires et/ou à l'injection de médicaments est en général contenu dans un boitier (Fig. 1 et Fig. 2) parallélipipèdique déjà décrit.

A titre d'example, la logique choisie pour cette version réalise les fonctions suivantes : marche avant coup par coup séparés par des périodes de relaxation, marche avant rapide et initialisation, retour arrière rapide irrévocable, commandé soit manuellement, soit automatiquement en cas de prolongation excessive de la durée de la période active.

Ces différentes fonctions sont obtenues respectivement (Fig. 5) :

– pour la marche avant coup par coup grâce à la charge à intensité constante d'un condensateur qui, couplé à un thyristor à gâchette d'anode constitue un relaxateur (29) qui initie la rotation du moto-réducteur (2-1) par amorçage d'un thyristor. L'arrêt du moteur est obtenu par la fermeture d'un contacteur disrupteur (10) sensible à l'aimant tournant (9) à travers l'enveloppe guide (5), ladite fermeture provoquant l'extinction du thyristor (31) par court-circuit suivie d'une ouverture qui supprime l'alimentation des bases de deux transistors complémentaires montés en émetteur commun dont l'un met alors l'induit du motoréducteur (2-1) en court-circuit.

– pour la marche avant rapide et initialisation, par un contacteur-poussoir (14) manuel qui, actionné, alimente par exemple la base du transistor de commande du motoréducteur (2-1). Une variante de cette fonction peut-être obtenue par un contacteur qui augmente fortement le courant de charge du condensateur horloge de relaxateur principal (29) :

– pour le retour arrière par excitation du relais inverseur soit manuellement (14) soit à l'aide d'un relaxateur auxiliaire (30). L'alimentation du moteur est alors partiellement dérivée sur une alarme sonore. La période de relaxation dudit relaxateur est choisie de manière à dépasser suffisamment la durée de délivrance d'un bolus sous pression normale ; lorsque, au contraire, cette durée est excessive par entrave à l'avancement du chariot (6) ce deuxième relaxateur (30) bascule le relais inverseur de marche via un thyristor. Cette fonction remplace à la fois un dispositif de détection d'emballement par suppression accidentelle des relaxations et la fin de course avant. La fin de course arrière est assurée par un interrupteur (20) sensible au champ magnétique.

La détermination de la force maximum exercée par l'ensemble motoréducteur (2); vis-sans-fin (3) ; chariot (6) et pion pousse-piston (7) sur le piston (21) de la seringue (19) est obtenue au moyen d'un contrôle de l'intensité traversant le motoréducteur (2-1). Le réglage de l'intensité à la valeur maximale choisie comme correspondant à une pression excessive est assurée approximativement par une résistance ballast en série avec le motoréducteur (2) et complétée par un réglage fin au moyen d'une résistance limitant le courant base du transistor monté en émetteur commun qui alimente le motoréducteur.

A titre de variante et non limitativement les contacts et/ou inverseur chargés de déterminer les fins de course avant et arrière peuvent être remplacés par un compteur-décompteur des tours positifs et négatifs de la vis-sans-fin de telle sorte que le nombre stocké dans ledit compteur-décompteur soit représentatif de la position du chariot entre lesdites fins de course. Ce mode présente l'avantage de pouvoir faire varier aisément la position géométique desdites fins de course et, ce faisant, d'adapter la course du chariot à la course du piston de la seringue.

Lorsque l'alimentation électrique est assurée par une batterie d'accumulateurs Cadmium-Nickel, on peut avantageusement munir la portière arrière (26) d'une douille (27) qui, reliée à un chargeur lui même branché sur le secteur, permet la recharge de la batterie d'accumulateurs sans démontage ni ouverture de son logement (28).

Avantageusement pour surveiller l'état de décharge de la batterie on peut réaliser un dispositif accessoire (Fig. 7) comportant une diode électroluminescente, un micro relaxateur, une diode Zeener et une résistance shunt.

Le dispositif peut être soit intégré dans le boitier, soit dans le chargeur (on le complète alors d'un interrupteur), ou encore être réalisé séparément et consulté après enfichage dans la douille de charge (27) de la batterie.

**Revendications**

1. Dispositif pousse-seringue monocourse électromécanique alimenté par batterie rechargeable, portable et pour traitement ambulatoire, à autonomie de plusieurs heures, comprenant un boîtier parallèlipipèdique, un ensemble électromécanique composé d'un moteur (2) à courant continu couplé à un réducteur (1), une vis-sans-fin (3) entrainant un chariot (6), et un ensemble de commande électronique, caractèrisé en ce que la contention de la seringue (19) est assurée par une pince de section trapèzoïdale réalisée par les expansions (16) adéquatement formées des faces latérales du boîtier parallèlipipèdique rectangle dont la face supérieure libre est fermée par la face supérieure d'une enveloppe-guide (5) quasi étanche et de section polyédrique contenant l'ensemble électro-mécanique, et en ce que le charriot est glissant à l'intérieur de l'enveloppe guide et de section homologue à celle-ci, et en ce que l'élasticité de ladite pince est renforcée par la présence de deux bandeaux portées par les faces latérales du boîtier, et en ce que l'ensemble électro-mécanique est monté flottant à l'intérieur de l'enveloppe guide (5) et composé d'un moteur (2) à courant continu sans fer tournant à excitation par aimant permanent de faible inertie et d'un réducteur (1) à haut rendement , et en ce que la vis-sans-fin (3) est fixée directement et coaxialement à l'arbre de sortie du réducteur (1) et libre de tout palier en bout, et en ce que les différentes fonctions du dispositif sont contrôlées par un ensemble électronique de programmation et d'asservissement tel que l'angle de rotation de la vis-sans-fin (3) correspondant à un volume prédéterminé est contrôlé par un aimant bipolaire (9) tournant en synchronisme avec la vis sans-fin (3) agissant à distance à travers l'enveloppe-guide (5) sur un disrupteur (10), que la pression du fluide contenu dans la seringue (19) est contrôlée par l'évaluation de la force de réaction exercée par le piston (21) de la seringue (19) sur le chariot (6) au moyen du contrôle de l'intensité traversant l'induit du motoréducteur (2-1), que le retour en position de départ du chariot (6) est assuré par l'inversion du sens de rotation du 5 motoréducteur (2-1) alimenté sous tension maximale jusqu'a ce que le chariot (6) ait atteint ladite position de départ, que le passage à la position de départ interrompt l'alimentation de tous les circuits électriques, à l'exception d'un unique circuit destiné à une initiation de la marche avant, et en ce que une prise de charge (14) accessible de l'extérieur permet la recharge des batteries sans démontage et/ou le contrôle de l'état de décharge de la batterie au moyen d'un dispositif externe.

2. Dispositif selon la revendication (1) caractèrisé en ce que le signal d'initiation de la rotation de la vis-sans-fin (3) correspondant à la délivrance de la dose élémentaire est obtenu par la décharge d'un condensateur attaquant un thyristor commandant deux transistor complémentaires montés en émetteur commun.

3. Dispositif selon la revendication (2) caractèrisé en ce que l'angle de rotation élémentaire est déterminé par un codeur optique ou à ultrason lié mécaniquement à la vis-sans-fin (3).

4. Dispositif selon l'une quelconque des revendications précédentes caractèrisé en ce que un compteur-décompteur des tours positifs et négatifs de la vis-sans-fin (3) permet la connaissance instantanée de la position géomètrique du chariot (6).

5. Dispositif selon la revendication (4) caractèrisé en ce que la fin de course avant est déterminée par la mise à zéro du compteur-décompteur lorsque le chariot (6) est dans la position choisie et la fin de course arrière par la mémorisation du nombre affiché par le compteur-décompteur lorsque le chariot (6) a atteint cette deuxième position choisie.

6. Dispositif selon l'une quelconque des revendications précédentes caractèrisé en ce que l'impulsion de commande du thyristor est fournie par un programmateur.

7. Dispositif selon la revendication (6) caractèrisé en ce que le programmateur est une horloge déterminant les périodes de relaxation.

8. Dispositif selon la revendication (7) caractèrisé en ce que le programmateur est lui-même asservi à une grandeur externe traduite en courant électrique.

9. Dispositif selon la revendication (8) caractèrisé en ce que la grandeur menante est la concentration plasmatique en glucose.

10. Dispositif selon l'une quelconque des revendications précédentes caractèrisé en ce que le franchissement d'une valeur prédéterminée par l'intensité du courant électrique traversant l'induit du motoréducteur (2-1) fait basculer un relais qui inverse le sens de rotation dudit motoréducteur.

11. Dispositif selon la revendication (10) caractèrisé en ce que l'inversion du sens du motoréducteur (2-1) est couplé à un signal sonore et/ou lumineux.

12. Dispositif selon l'une quelconque des revendications précédentes caractèrisé en ce que la fin de course aller est précédée par une alarme rythmée lorsqu'il ne reste plus qu'un nombre prédéterminé de doses élémentaires.

13. Dispositif selon l'une quelconque des revendications précédentes caractèrisé en ce que le retour du chariot (6) en position de départ est obtenu par inversion des polarités du motoréducteur (2-1) au moyen d'un double inverseur à commande manuelle et/ou automatique, ledit inverseur étant remis en position marche avant par un interrupteur (22) de fin de course retour.

14. Dispositif selon l'une quelconque des revendications précédentes caractèrisé en ce que l'alimentation électrique est assuré par une batterie d'éléments Cadmium-Nickel étanches.

15. Dispositif selon l'une quelconque des revendications précédentes caractèrisé en ce que l'opérateur est averti de la nécessité de recharger les batteries par une alarme optique clignotante.

**Patentansprüche**

1. Elektromechanische Vorrichtung zum einwegigen Ausdrücken von Injektionsspritzen, gespeist durch eine wiederaufladbare Batterie, wobei sie tragbar und für eine ambulante Behandlung vorgesehen ist, mit einer Unabhängigkeit von mehreren Stunden, mit einem parallelepipedischen Gehäuse, einer elektromechanischen Anordnung, die aus einem Motor (2) mit kontinuierlichem Strom, der mit einem Getriebe (1) verbunden ist, einer Schnecke (3), die einen Schlitten (6) antreibt und einer elektronischen Steueranordnung gebildet ist, **dadurch gekennzeichnet**, daß die Retention oder Anspannung der Spritze (19) sichergestellt wird durch eine Zange von trapezförmigem Querschnitt, die durch die Ausdehnungen (16) gebildet wird, die entsprechend aus seitlichen Seiten des rechteckigen parallelepipedischen Gehäuses gebildet sind, dessen freie Oberseite durch die Oberseite einer Führungsumhüllung (5) verschlossen ist, die quasi dicht und von einem Vieleckquerschnitt ist, enthaltend die elektromechanische Anordnung, und daß der Schlitten im Inneren der Fürungsumhüllung gleitbar und von ähnlichem Querschnitt ist, und daß die Elastizität der Zange durch das Vorhandensein von zwei Bändern verstärkt wird, die von den seitlichen Seiten des Gehäuses getragen werden, und daß die elektromechanische Anordnung schwimmend im Inneren der Fürungsumhüllung (5) angeordnet ist und aus einem Motor (2) mit kontinuierlichem Strom ohne Eisen gebildet ist, der durch Permanentmagnetanregung von schwacher Trägheit zum Drehen angeregt wird, und aus einem Untersetzungsgetriebe (1) mit hohem Wirkungsgrad gebildet wird, und daß die Schnecke (3) direkt und koaxial zur Ausgangswelle des Untersetzungsgetriebes (1) befestigt ist und von jeglichem Lagerarmende frei ist, und daß die verschiedenen Funktionen der Vorrichtung gesteuert werden durch eine elektronische Programmier- und Verarbeitungsanordnung, derart, daß der Drehwinkel der Schnecke (3), der einem vorbestimmten Volumen entspricht, durch einen bipolaren Magneten (9) gesteuert wird, der sich synchron mit der Schnecke (3) dreht, die beabstandet durch die Fürungsumhüllung (5) auf einem Durchschlag (10) wirkt, daß der Druck der in der Spritze (19) enthaltenen Flüssigkeit durch Bewertung der Reaktionskraft gesteuert wird, die durch den Kolben (21) der Spritze (19) auf den Schlitten (6) ausgelebt wird, mittels der Kontrolle der Intensität, die den Läufer der Motor-Getriebeeinheit (2-1) durchquert, daß das Zurückkehren in die Ausgangsposition des Schlittens (6) sichergestellt wird durch Umkehren der Drehrichtung der Motor-Getriebeeinheit (2-1) gespeist unter maximaler Spannung bis der Schlitten (6) die Ausgangsposition erreicht hat, daß das Zuführen zur Ausgangsposition die Speisung bzw. Erregung aller elektrischer Schaltkreise unterbricht mit Ausnahme eines einzigen Schaltkreises, der dazu bestimmt ist, die Vorwärtsbewegung auszulösen, und daß eine Steckdose (14), die von außen zugänglich ist, die Wiederaufladung der Batterien ohne Demontage und-/oder die Kontrolle des Entladungszustandes der Batterie mit Hilfe einer äußeren Vorrichtung erlaubt.

2. Vorrichtung nach Anspruch 1, **dadurch gekennzeichnet**, daß das Ausgangssignal der Drehung der Schnecke (3), die dem Liefern der elementaren Dosis entspricht, erhalten wird durch Entladen eines Kondensators, der einen Thyristor beaufschlagt, der zwei komplementäre Transistoren steuert, die als gemeinsamer Emitter angeordnet sind.

3. Vorrichtung nach Anspruch 2, **dadurch gekennzeichnet**, daß der elementare Drehwinkel bestimmt wird durch einen optischen oder Ultraschall-Kodierer, der mechanisch mit der Schnecke (3) verbunden ist.

4. Vorrichtung nach einem der vorhergehenden Ansprüche, **dadurch gekennzeichnet**, daß ein Auf- und Abzähler der positiven und negativen Drehungen der Schnecke (3) das augenblickliche Wissen der geometrischen Position des Schlittens (6) erlaubt.

5. Vorrichtung nach Anspruch 4, **dadurch gekennzeichnet**, daß das Ende der vorderen Bahn bestimmt ist durch das auf Nullstellen des Zählers, wenn der Schlitten (6) in der gewählten Position ist und das Ende der hinteren Bahn durch das Speichern der angezeigten Zahl durch den Zähler bestimmt ist, wenn der Schlitten (6) jede zweite ausgewählte Stellung erreicht hat.

6. Vorrichtung nach einem der vorhergehenden Ansprüche, **dadurch gekennzeichnet**, daß der Steuerimpuls des Thyristors durch ein Programmschaltwerk geliefert wird.

7. Vorrichtung nach Anspruch 6, **dadurch gekennzeichnet**, daß das Programmschaltwerk eine Uhr ist, die die Entspannungsperioden bestimmt.

8. Vorrichtung nach Anspruch 7, **dadurch gekennzeichnet**, daß das Progammschaltwerk selbst von einer äußeren Größe abhängt, die in elektrischen Strom übersetzt ist.

9. Vorrichtung nach Anspruch 8, **dadurch gekennzeichnet**, daß die treibende Größe die Glukose-Plasma-Konzentration ist.

10. Vorrichtung nach einem der vorhergehenden Ansprüche, **dadurch gekennzeichnet**, daß das Überschreiten eines vorbestimmten Werts durch die Intensität des elektrischen Stroms, der durch den Rufer der Motor-Getriebeeinheit (2-1) geht, ein Relais hinund herbewegt, das die Drehrichtung der Motor-Getriebeeinheit umdreht.

11. Vorrichtung nach Anspruch 10, **dadurch gekennzeichnet**, daß die Umdrehung der Richtung der Motor-Getriebeeinheit (2-1) mit einem Schall- und/oder Leuchtsignal verbunden ist.

12. Vorrichtung nach einem der vorhergehenden Ansprüche, **dadurch gekennzeichnet**, daß dem Ende der Vorwärtsbewegung ein rhythmischer Alarm vorhergeht, wem nur noch eine vorbestimmte Anzahl von Elementardosen verbleibt.

13. Vorrichtung nach einem der vorhergehenden Ansprüche, **dadurch gekennzeichnet**, daß die Rückkehr des Schlittens (6) in die Ausgangsstellung durch Umkehrung der Polaritäten der Motor-Getriebeeinheit (2-1) mit Hilfe eines doppelten Umkehrers mit manueller und/oder automatischer Steuerung erreicht wird, wobei der Umkehrer mit Vorwärtsbewegungsstellung durch den Schalter (22) des Endes der Rückwärtsbewegung zurückgesetzt wird.

14. Vorrichtung nach einem der vorhergehenden Ansprüche, **dadurch gekennzeichnet**, daß die elektrische Versorgung durch eine Batterie mit dichten Cadmium-Nickel-Elementen sichergestellt ist

15. Vorrichtung nach einem der vorhergehenden Ansprüche, **dadurch gekennzeichnet**, daß die Bedienungsperson von der Notwendigkeit, die Batterien wieder aufzuladen, durch einen optischen blinkenden Alarm aufmerksam gemacht wird.

## Claims

1. Electro-mechanical single-stroke syringe-driver device supplied by rechargeable battery, portable and for ambulatory treatment with independence of several hours, comprising a parallelepipedic casing, an electro-mechanical assembly composed of a D.C. motor (2) coupled to a reduction gear (1), an endless screw (3) driving a carriage (6), and an electronic control assembly, characterized in that the holding of the syringe (19) is ensured by a grip of trapezoidal section which is performed by the adequately formed expansions (16) of the lateral faces of the rectangular parallelepipedic casing of which the upper free face is closed by the upper face of a virtually tight guide envelope (5) of polyhedral section containing the electro-mechanical assembly, and in that the carriage is sliding inside the guide envelope and of homologous section to the latter, and in that the elasticity of said grip is reinforced by the presence of two bands borne by the lateral faces of the casing, and in that the electro-mechanical assembly is mounted in floating manner inside the guide envelope (5) and composed of a D.C. motor (2) without iron rotating with energization by permanent magnet of low inertia, and of a reduction gear (1) with high yield, and in that the endless screw (3) is fixed directly and coaxially to the driven shaft of the geared motor (1) and is free of any additional bearing, and in that the different functions of the device are controlled by a servo-controlled and programmed electronic assembly such as the angle of rotation of the endless screw (3) corresponding to a predetermined volume is controlled by a bipolar magnet (9) rotating in synchronism with the endless screw (3) acting remotely through the guide envelope (5) on a disruptor (10), and in that the pressure of the fluid contained in the syringe (9) is controlled by the assessment of the force of reaction exerted by the piston (21) of the syringe (19) on the carriage (6) by means of the control of the intensity passing through the armature of the geared motor (2-1), and in that the return into starting position of the carriage (6) is ensured by the reversal of the direction of rotation of the geared motor (2-1) supplied with maximum voltage until the carriage (6) has attained said starting position, and in that the passage to the starting position interrupts the supply of all the electrical circuits, with the exception of a single circuit intended for initiation of forward operation, and in that a charging tap (14) accessible from the outside allows recharging of the batteries without dismantling and/or control of the state of discharge of the battery by means of an outside device.

2. Device according to claim 1, characterized in that the signal for initiation of rotation of the endless screw (3) corresponding to the delivery of the elementary dose is obtained by the discharge of a capacitor attacking a thyristor controlling two complementary transistors mounted in common emitter.

3. Device according to claim 2, characterized in that the angle of elementary rotation is determined by an optical or ultra-sonic coder mechanically connected to the endless screw (3).

4. Device according to any one of the preceding claims, characterized in that a reversible counter four counting the positive and negative turns of the endless screw (3) makes it possible to know the geometrical position of the carriage (6) instantaneously.

5. Device according to claim 4, characterized in that the forward end-of-stroke is determined by the return to zero of the reversible counter when the carriage (6) is in the chosen position and the rearward end-of-stroke by the memorization of the number displayed by the reversible counter when the carriage (6) has attained this second chosen position.

6. Device according to any one of the preceding claims, characterized in that the pulse for controlling the thyristor is furnished by a programmer.

7. Device according to claim 6, characterized in that the programmer is a clock determining the periods of relaxation.

8. Device according to claim 7, characterized in that the programmer is itself servo-controlled by an outside magnitude translated into electrical current.

9. Device according to claim 8, characterized in that the driving magnitude is the plasmatic concentration in glucose.

10. Device according to any one of the preceding claims, characterized in that the exceeding of a predetermined value by the intensity traversing the armature of the geared motor (2-1) causes a relay to tip which reverses the direction of rotation of said geared motor.

11. Device according to claim 10, characterized in that the reversal of the directtion of the geared motor (2-1) is coupled to a sound and/or light signal.

12. Device according to any one of the preceding claims, characterized in that the forward end-of-stroke is preceded by a rhythmic alarm when only a predetermined number of elementary doses remain.

13. Device according to any one of the preceding claims, characterized in that the return of the carriage (6) into starting position is obtained by reversal of the polarities of the geared motor (2-1) by means of a double inverter with manual and/or automatic control, said inverter being returned into forward operation position by a return end-of-stroke switch (22).

14. Device according to any one of the preceding claims, characterized in that electrical supply is ensured by a battery of sealed cadmium-nickel elements.

15. Device according to any one of the preceding claims, characterized in that the operator is warned of the necessity of recharging the batteries by a flashing optical alarm.

FIG. 1

FIG. 2

FIG. 3

9

FIG.4

FIG.5

FIG.6

FIG.7